(19) **Europäisches Patentamt European Patent Office Office européen des brevets**

(11) **EP 4 152 335 B1**

(12) **FASCICULE DE BREVET EUROPEEN**

(45) Date de publication et mention de la délivrance du brevet:
**18.02.2026 Bulletin 2026/08**

(21) Numéro de dépôt: **22194899.5**

(22) Date de dépôt: **09.09.2022**

(51) Classification Internationale des Brevets (IPC):
**G16C 20/20** *(2019.01)* **G01N 33/18** *(2006.01)*

(52) Classification Coopérative des Brevets (CPC):
**G16C 20/20;** G01N 33/1826

(54) **PROCÉDÉ DE CARACTÉRISATION NON CIBLÉE D'UNE SOLUTION COMPRENANT UNE PLURALITÉ DE SOLUTÉS**

VERFAHREN ZUR NICHT ZIELGERICHTETEN CHARAKTERISIERUNG EINER LÖSUNG, DIE EINE VIELZAHL VON GELÖSTEN STOFFEN ENTHÄLT

METHOD FOR NON-TARGETED CHARACTERISATION OF A SOLUTION COMPRISING A PLURALITY OF SOLUTES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.09.2021 FR 2109802**

(43) Date de publication de la demande:
**22.03.2023 Bulletin 2023/12**

(73) Titulaire: **Commissariat à l'Energie Atomique et aux Energies Alternatives**
**75015 Paris (FR)**

(72) Inventeurs:
• **DAGNELIE, Romain**
**91300 MASSY (FR)**
• **DISDIER, Zoé**
**92210 SAINT-CLOUD (FR)**

(74) Mandataire: **Santarelli**
**Tour Trinity**
**1 bis Place de la Défense**
**92400 Courbevoie (FR)**

(56) Documents cités:
• **MILMAN BORIS L ET AL: "The chemical space for non-target analysis", TRAC TRENDS IN ANALYTICAL CHEMISTRY, vol. 97, 3 October 2017 (2017-10-03), pages 179 - 187, XP085268092, ISSN: 0165-9936, DOI: 10.1016/J.TRAC.2017.09.013**

**Description**

**Domaine technique**

L'invention se rapporte au domaine de la caractérisation de solutions.

Plus spécifiquement, elle se rapporte à un procédé de caractérisation non ciblée d'une solution comprenant une pluralité de solutés qui est basé sur une séquence de partages, ou extractions, de ces solutés entre deux phases liquides non miscibles, respectivement aqueuse et organique.

Ce procédé trouve application dans tous les domaines où l'on peut souhaiter caractériser une solution aqueuse ou organique sans avoir à en déterminer la composition.

Ainsi, il peut notamment trouver application pour :

- caractériser un effluent agricole ou industriel en vue de sa gestion ultérieure, en particulier un effluent de composition complexe comprenant, par exemple, des solutés issus d'une dégradation biotique ou abiotique (chimique, radiolytique ou autre) ;
- évaluer la qualité d'un produit naturel ou industriel, en particulier de nature alimentaire (et, par la même, la fiabilité des procédés mis en œuvre pour sa culture, sa collecte ou sa fabrication, sa conservation et sa distribution) ;
- évaluer la qualité d'une eau telle qu'une eau issue d'une station d'épuration (et, par la même, la fiabilité du procédé d'épuration utilisé), une eau de mer ou l'eau d'un cours d'eau, d'un plan d'eau, naturel ou artificiel, ou d'une nappe phréatique ; ou encore
- évaluer le degré de pureté de substances de synthèse, marquées ou non, et caractériser la quantité et les propriétés des éventuelles impuretés que comprennent ces substances.

**État de la technique antérieure**

Il existe de nombreuses méthodes permettant de caractériser une solution.

Parmi ces méthodes, on distingue des méthodes dites directes ou « ciblées » et des méthodes dites « non ciblées ».

Comme leur nom l'indique, les méthodes ciblées visent à détecter la présence de solutés connus dans la solution et, généralement, à en déterminer la concentration. Ces méthodes comprennent typiquement une étape préalable de séparation des solutés, par exemple, par chromatographie.

Bien qu'appliquées dans de nombreux domaines dont, notamment, le suivi et la surveillance de milieux naturels, les méthodes ciblées présentent un intérêt limité lorsque la solution devant être caractérisée est de composition complexe et/ou comprend des solutés inconnus, ce qui est notamment le cas de solutions comprenant des solutés résultant d'une dégradation bactérienne, chimique, radiolytique ou autre de composés organiques.

C'est la raison pour laquelle ont été proposées des méthodes non ciblées qui visent à détecter la présence dans une solution d'une gamme aussi large que possible de solutés, y compris des solutés jamais étudiés et, donc, encore inconnus. À titre d'exemple de méthodes non ciblées, on peut citer celle qui est basée sur l'utilisation de la chromatographie liquide couplée à la spectroscopie de masse haute résolution (ou LC-HRMS) et qui a notamment été mise en œuvre par Mondeguer et al. (Spectra Analyse 2012, 284, 25-33) pour établir un profilage de type métabolomique des toxines bioactives du phytoplancton qui s'accumulent dans les coquillages, et par Fischer et al. (Trends in Analytical Chemistry 2021, 136, 116188) pour suivre la présence de pesticides dans des aliments. On peut également citer les travaux de Milman et Zhurkovich (Trends in Analytical Chemistry 2017, 97, 179-187) sur l'utilisation des bases de données chimiques générales et spécialisées dans l'analyse des données obtenues par des méthodes non ciblées.

Bien que certaines méthodes non ciblées parviennent à un stade de développement leur permettant de bénéficier d'une validation ou d'une accréditation, elles restent généralement difficiles à mettre en œuvre et insuffisantes lorsqu'il s'agit de caractériser des solutions de composition inconnue particulièrement complexe.

**Exposé de l'invention**

L'invention vise justement à proposer un procédé de caractérisation non ciblée d'une solution - dite solution $S_0$ dans ce qui suit - qui est simple à mettre en œuvre et qui est applicable à toute solution, aqueuse ou organique, de composition inconnue et ce, quelle que soit la complexité de cette composition.

Ce procédé comprend au moins les étapes consistant à :

a) soumettre la solution $S_0$ à une séquence de N rangs i de partages, N étant supérieur ou égal à 2 et i allant de 1 à N, les rangs i de partages comprenant :

- pour i=1, le mélange d'un échantillon de la solution $S_0$ avec un volume d'une solution organique $S_{org}$ ou aqueuse $S_{aq}$ non miscible à la solution $S_0$ pour obtenir, après séparation du mélange en deux phases, une phase aqueuse $\varphi(i=1,aq)$ et une phase organique $\varphi(i=1,org)$ ; et
- pour i=2 à N :

  * le mélange de chaque phase aqueuse $\varphi(i-1,aq)$ avec un volume de la solution $S_{org}$ pour obtenir, après séparation du mélange en deux phases, une phase aqueuse $\varphi(i,aq)$ et une phase organique $\varphi(i,org)$ ;
  * le mélange de chaque phase organique $\varphi(i-1,org)$ avec un volume de la solution $S_{aq}$ pour obtenir, après séparation du mélange en deux phases, une phase aqueuse $\varphi(i,aq)$ et une phase organique $\varphi(i,org)$ ;

b) soumettre toutes les phases aqueuses et organiques obtenues au rang N à une mesure d'une grandeur X extensive et conservative pour obtenir un vecteur expérimental $\vec{V}exp$ comprenant $2^N$ valeurs de X mesurées ;
c) calculer des vecteurs théoriques $\vec{V}$théor$(D_k)$ pour y valeurs $D_k$ de coefficient de partage avec y supérieur ou égal à 2 et k allant de 1 à y, chaque vecteur théorique comprenant $2^N$ valeurs de X calculées pour les phases aqueuses et organiques obtenues au rang N et pour l'une des y valeurs $D_k$ ; et
d) réaliser un ajustement paramétrique entre les vecteurs $\vec{V}$théor$(D_k)$ et le vecteur $\vec{V}exp$ pour obtenir une répartition des solutés de la solution $S_0$, qui contribuent aux $2^N$ valeurs de X mesurées, en fonction des y valeurs $D_k$.

Ainsi, le procédé de l'invention est basé sur :

- la réalisation d'une séquence de N rangs successifs d'extractions liquide-liquide, cette séquence conduisant à un total de $[2^{(N+1)}-1]$ phases différentes dont $2^N$ phases sont issues du dernier rang N de partages (étape a) du procédé),
- l'acquisition de données expérimentales par la mesure d'une grandeur X dans les $2^N$ phases issues du dernier rang N de partages de ladite séquence (étape b) du procédé), et
- un traitement mathématique des données expérimentales ainsi acquises, ce traitement permettant de déterminer la répartition des solutés de la solution $S_0$, qui contribuent aux valeurs de la grandeur X obtenues expérimentalement, en fonction de différentes valeurs d'hydrophilie/lipophilie (étapes c) et d) du procédé).

Compte-tenu que le caractère hydrophile/lipophile de solutés est intrinsèque à ces solutés et représente une information pertinente sur un certain nombre de leurs propriétés comme, par exemple, leur solubilité dans un milieu tel que l'environnement et, donc, leur devenir dans ce milieu, leur toxicité, leur bioaccumulation, etc., le procédé permet d'obtenir une information utile sur la solution $S_0$ mais sans avoir à en déterminer la composition exacte ni à identifier les solutés qu'elle comprend.

Conformément à l'invention, N est typiquement compris entre 2 et 20 et, de préférence, entre 3 et 10.

La solution $S_0$ est, de préférence, une solution aqueuse, auquel cas la solution $S_{org}$ est typiquement un solvant organique non miscible à l'eau, éventuellement préalablement hydraté (c'est-à-dire pré-équilibré par contact avec de l'eau), tandis que la solution $S_{aq}$ est une solution aqueuse qui présente, de préférence, une force ionique et un pH proches de ceux de la solution $S_0$ et qui peut donc comprendre, pour ce faire, un sel de fond du type KCl ou $NaClO_4$ et/ou un tampon pH en proportions appropriées dans de l'eau, de préférence distillée.

Le solvant organique non miscible à l'eau est, par exemple, un alcool en C8 à C12 (tel que le n-octanol, n-décanol ou le n-dodécanol), un alcane en C5 à C16 (tel que le n-hexane, le n-heptane ou le n-octane), le cyclohexane, un hydrocarbure aromatique (tel que le benzène, le toluène, l'éthylbenzène ou le xylène), le chloroforme, etc. Parmi ceux-ci, préférence est donnée au n-octanol.

En variante, la solution $S_0$ peut également être une solution organique, auquel cas la solution $S_{aq}$ est typiquement de l'eau, de préférence distillée, tandis que la solution $S_{org}$ peut être le même solvant organique que celui que comprend la solution $S_0$ dans le cas où l'on sait quel est le solvant organique de la solution $S_0$, ou, à défaut, l'un des solvants organiques précédemment cités, éventuellement préalablement hydraté, préférence étant donnée, là également, au n-octanol.

Conformément à l'invention, la grandeur X peut être toute grandeur permettant de cibler une propriété d'intérêt présentée par les solutés ou certains solutés de la solution $S_0$ pour autant que cette grandeur soit extensive et conservative.

Par grandeur « extensive », on entend une grandeur dont la somme des valeurs pour deux systèmes disjoints est égale à la valeur de la grandeur pour la réunion des deux systèmes, ce qui signifie concrètement, dans le cadre du procédé de l'invention, que, pour une phase de composition donnée, la grandeur X est proportionnelle au volume de cette phase.

Par grandeur « conservative », on entend une grandeur qui reste constante pour un système isolé n'échangeant pas de matière avec l'extérieur, ce qui signifie concrètement, dans le cadre du procédé de l'invention, que la grandeur X se conserve à chaque partage et qu'en l'absence de tout prélèvement de matière, la somme des valeurs de X dans les phases aqueuses et organiques obtenues au rang N de partages est égale à la valeur de X dans la solution $S_0$.

Ainsi, la grandeur X peut notamment être une quantité de matière (molaire, massique ou autre), une concentration (si la quantité de matière est rapportée à une unité de volume constant), une absorbance, une fluorescence, une radioactivité ou une opacité.

Conformément à l'invention, chaque vecteur $\vec{V}$théor($D_k$) peut être défini en appliquant :

- au partage de l'échantillon de la solution $S_0$, les équations (1a) et (1b) suivantes :

$$X_{(i=1,aq)}(D_k) = X_0 \times \frac{1}{\left(1 + D_k \times \frac{V_{(0,org)}}{V_{(0,aq)}}\right)} \qquad (1a)$$

$$X_{(i=1,org)}(D_k) = X_0 \times \frac{D_k \times \frac{V_{(0,org)}}{V_{(0,aq)}}}{\left(1 + D_k \times \frac{V_{(0,org)}}{V_{(0,aq)}}\right)} \qquad (1b)$$

- aux partages des phases φ(i,aq), les équations (2a) et (2b) suivantes :

$$X_{(i+1,aq)}(D_k) = X_{(i,aq)}(D_k) \times \frac{1}{\left(1 + D_k \times \frac{V_{(i,org)}}{V_{(i,aq)}}\right)} \qquad (2a)$$

$$X_{(i+1,org)}(D_k) = X_{(i,aq)}(D_k) \times \frac{D_k \times \frac{V_{(i,org)}}{V_{(i,aq)}}}{\left(1 + D_k \times \frac{V_{(i,org)}}{V_{(i,aq)}}\right)} \qquad (2b)$$

- aux partages des phases φ(i,org), les équations (3a) et (3b) suivantes :

$$X_{(i+1,aq)}(D_k) = X_{(i,org)}(D_k) \times \frac{1}{\left(1 + D_k \times \frac{V_{(i,org)}}{V_{(i,aq)}}\right)} \qquad (3a)$$

$$X_{(i+1,org)}(D_k) = X_{(i,org)}(D_k) \times \frac{D_k \times \frac{V_{(i,org)}}{V_{(i,aq)}}}{\left(1 + D_k \times \frac{V_{(i,org)}}{V_{(i,aq)}}\right)} \qquad (3b)$$

dans lesquelles :

$X_{(i=1,aq)}$ est la valeur de X attendue pour des solutés présentant la valeur $D_k$ et pour la phase φ(i=1,aq),
$X_{(i=1,org)}$ est la valeur de X attendue pour des solutés présentant la valeur $D_k$ et pour la phase φ(i=1,org),
$X_0$ est la valeur de X mesurée dans la solution $S_0$,
$V_{(o,org)}$ est le volume de l'échantillon de solution $S_0$ si la solution $S_0$ est une solution organique ou le volume de solution $S_{org}$ utilisé pour partager l'échantillon de la solution $S_0$ si la solution $S_0$ est une solution aqueuse,
$V_{(0,aq)}$ est le volume de l'échantillon de solution $S_0$ si la solution $S_0$ est une solution aqueuse ou le volume de solution $S_{aq}$ utilisé pour partager l'échantillon de la solution $S_0$ si la solution $S_0$ est une solution organique,
$X_{(i+1,aq)}$ est la valeur de X attendue pour des solutés présentant la valeur $D_k$ et pour une phase φ(i+1,aq) issue du partage d'une phase φ(i,aq) ou d'une phase φ(i,org),
$X_{(i+1,org)}$ est la valeur de X attendue pour des solutés présentant la valeur $D_k$ et pour une phase φ(i+1,org) issue du partage d'une phase $\varphi(i,aq)$ ou d'une phase $\varphi(i,org)$,

$X_{(i,aq)}$ est la valeur de X attendue pour des solutés présentant la valeur $D_k$ et pour la phase $\varphi(i,aq)$ partagée,

$X_{(i,org)}$ est la valeur de X attendue pour des solutés présentant la valeur $D_k$ et pour la phase $\varphi(i,org)$ partagée,

V(i,aq) est le volume de la phase $\varphi(i,aq)$ partagée ou le volume de solution $S_{aq}$ utilisé pour partager la phase $\varphi(i,org)$,

V(i,org) est le volume de la phase $\varphi(i,org)$ partagée ou le volume de solution $S_{org}$ utilisé pour partager la phase $\varphi(i,aq)$,

moyennant quoi on obtient pour les phases aqueuses et organiques du rang N, $2^N$ valeurs de X attendues pour des solutés présentant la valeur $D_k$. Ces $2^N$ valeurs extensives de X forment un vecteur $\vec{V}$théor($D_k$).

L'ajustement paramétrique consiste, de préférence, à déterminer un ensemble de pourcentages $P_k$ en tant que coefficients de pondération tels que la somme des vecteurs $\vec{V}$théor($D_k$) pondérée par ces coefficients de pondération soit la plus proche possible du vecteur $\vec{V}$exp ou, en d'autres termes, tels que :

$$\vec{V}\text{exp} \approx \Sigma(P_k\,(\%) \times \vec{V}\text{théor}(D_k)).$$

Cet ajustement peut être réalisé par une méthode de régression conventionnelle telle que la méthode des moindres carrés, et ce, au moyen d'un logiciel du type Excel™ ou OriginLab™.

L'ajustement peut en outre comprendre un ajustement des valeurs de D et, donc, des vecteurs $\vec{V}$théor($D_k$) eux-mêmes. Le résultat de l'ajustement est, par exemple, exprimé sous la forme d'un histogramme représentant, sous la forme de pourcentages ($P_k$), la répartition des solutés de la solution $S_0$, qui contribuent aux $2^N$ valeurs de X mesurées, en fonction des y valeurs de coefficient de partage ($D_k$).

Conformément à l'invention, les solutés sont, de préférence, des solutés organiques.

D'autres caractéristiques et avantages de l'invention apparaîtront à la lecture du complément de description qui suit et qui est donné en référence aux figures annexées.

Il va de soi que ce complément de description n'est donné qu'à titre d'illustration de l'objet de l'invention et ne constitue en aucun cas une limitation de cet objet.

**Brève description des figures**

La figure 1A et la figure 1B illustrent, schématiquement et à titre d'exemple, l'utilité de soumettre deux solutions 1 et 2 de compositions différentes à une séquence de N rangs de partages pour leur caractérisation et, ainsi, la mise en évidence de leur différence de composition.

La figure 2 illustre, schématiquement et à titre d'exemple, les phases qui sont obtenues dans le cas où une solution $S_0$ est soumise à une séquence de 3 rangs de partages.

La figure 3 illustre, schématiquement et à titre d'exemple, un traitement mathématique des données expérimentales et du résultat obtenu par ce traitement mathématique.

La figure 4 illustre, schématiquement et à titre d'exemple, les résultats d'une expérimentation ayant été réalisée avec un mélange connu de trois solutés et ayant permis de valider le procédé de l'invention.

La figure 5 illustre, à titre d'exemple, les résultats de l'application du procédé de l'invention à l'évaluation du degré de pureté d'une substance commerciale, à savoir du bisphénol A marqué par du $^{14}$C, et à la caractérisation de la quantité et de la lipophilie d'une impureté présentée par cette substance.

**Exposé détaillé de modes de mise en œuvre préférés**

**1. Rappels théoriques** :

Le coefficient de partage d'un soluté A traduit la répartition de ce soluté entre deux phases immiscibles, respectivement aqueuse et organique, qui sont mises en contact l'une avec l'autre.

Il est typiquement défini par un rapport de concentrations, noté ci-après D(A), selon l'équation (4) :

$$D(A) = \frac{[A]_{org}}{[A]_{aq}} \qquad (4)$$

dans laquelle :

$[A]_{org}$ est la concentration molaire du soluté A dans la phase organique à l'équilibre, et
$[A]_{aq}$ est la concentration molaire du soluté A dans la phase aqueuse à l'équilibre.

Pour un soluté A qui se présente en solution sous plusieurs espèces (telle qu'un acide faible qui se dissocie en une base A⁻ et un proton), le coefficient de partage D(A) se définit par l'équation (5) :

$$D(A) = \frac{\sum [A_i]_{org}}{\sum [A_i]_{aq}} \quad (5)$$

dans laquelle :

$[A_i]_{org}$ est la concentration molaire de chaque espèce du soluté A dans la phase organique à l'équilibre,
$[A_i]_{aq}$ est la concentration molaire de chaque espèce du soluté A dans la phase aqueuse à l'équilibre.

Il est également possible de définir le coefficient de partage par un rapport en nombres de moles, noté ci-après D'(A), selon l'équation (6) :

$$D'(A) = \frac{n(A)_{org}}{n(A)_{aq}} = D(A) \frac{V_{org}}{V_{aq}} \quad (6)$$

dans laquelle :

$n(A)_{org}$ est le nombre de moles du soluté A présentes dans la phase organique à l'équilibre,
$n(A)_{aq}$ est le nombre de moles du soluté A présentes dans la phase aqueuse à l'équilibre,
$V_{org}$ est le volume de la phase organique, et
$V_{aq}$ est le volume de la phase aqueuse.

Pour un soluté A qui se présente en solution sous plusieurs espèces, l'équation (6) devient l'équation (7) :

$$D'(A) = \frac{\sum n(A_i)_{org}}{\sum n(A_i)_{aq}} \quad (7)$$

dans laquelle :

$n(A_i)_{org}$ est le nombre de moles de chaque espèce du soluté A présentes dans la phase organique à l'équilibre, et
$n(A_i)_{aq}$ est le nombre de moles de chaque espèce du soluté A présentes dans la phase aqueuse à l'équilibre.

Il est à noter que les coefficients de partage D et D' sont sans dimension et sont généralement exprimés sous la forme de leur logarithme en base dix, notés respectivement ci-après log D et log D'.
Le partage d'un soluté A en solution entre deux phases, respectivement aqueuse et organique, conduit aux bilans (8a), (8b), (9a) et (9b) suivants :

$$C_{aq} = C_0 \times \frac{1}{(1+D)} \quad (8a)$$

$$C_{org} = C_0 \times \frac{D}{(1+D)} \quad (8b)$$

$$n_{aq} = n_0 \times \frac{1}{(1+D')} \qquad (9a)$$

$$n_{org} = n_0 \times \frac{D'}{(1+D')} \qquad (9b)$$

dans lesquels :

$C_0$ est la concentration initiale du soluté A (ou la somme des concentrations initiales de ses espèces) dans la solution soumise au partage,

$C_{aq}$ est la concentration du soluté A (ou la somme des concentrations de ses espèces) dans la phase aqueuse issue du partage,

$C_{org}$ est la concentration du soluté A (ou la somme des concentrations de ses espèces) dans la phase organique issue du partage,

$n_0$ est le nombre de moles du soluté A (ou la somme des moles de ses espèces) initialement présentes dans la solution soumise au partage,

$n_{aq}$ est le nombre de moles du soluté A (ou la somme des moles de ses espèces) présentes dans la phase aqueuse issue du partage, et

$n_{org}$ est le nombre de moles du soluté A (ou la somme des moles de ses espèces) présentes dans la phase organique issue du partage.

## 2. Utilité d'une séquence de N rangs de partages et application au procédé de l'invention (étape a) du procédé) :

Lorsqu'une solution comprenant plusieurs solutés est soumise à un seul rang de partages, la mesure d'une grandeur X, telle qu'une concentration, dans les phases aqueuse et organique issues du premier rang de partages fournit une moyenne pondérée du coefficient de partage de chacun des solutés contribuant à cette grandeur.

En conséquence, pour une grandeur X donnée, il est possible d'obtenir des coefficients de partage identiques pour des solutions de compositions différentes si ces solutions sont soumises à un seul rang de partages.

Par contre, si l'on soumet ces solutions à un deuxième rang de partages, alors les coefficients de partage obtenus pour les phases issues du deuxième rang de partages permettent de différencier les solutions.

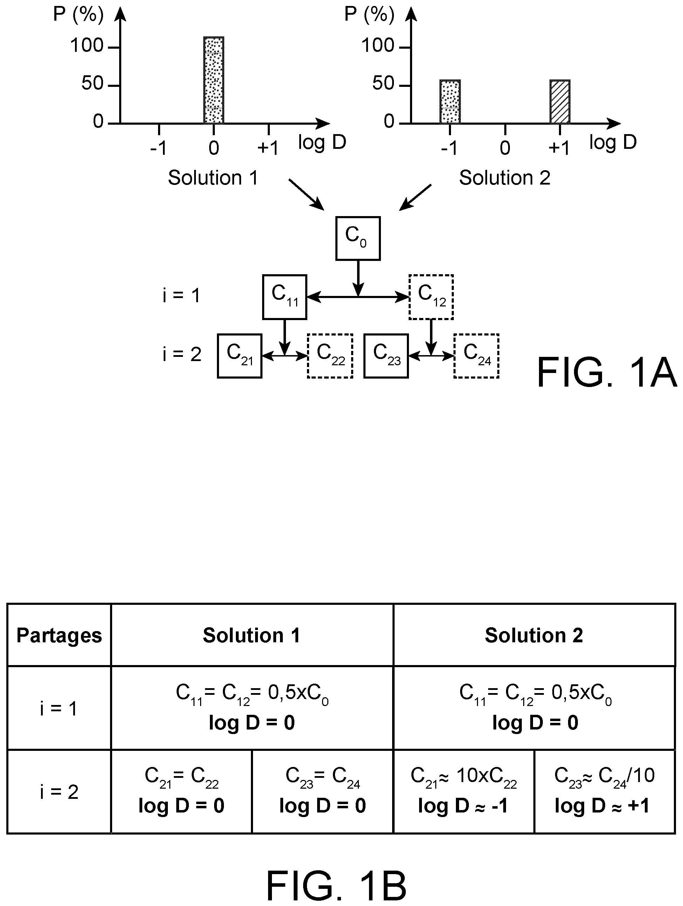

Ceci est illustré sur les figures 1A et 1B pour deux solutions dont l'une - dite solution 1 - ne comprend qu'un soluté qui est aussi hydrophile que lipophile (log D = 0) tandis que l'autre - dite solution 2 - comprend deux solutés en quantités équimolaires dont l'un est hydrophile (log D = -1) et l'autre lipophile (log D = +1), et que l'on soumet à deux rangs de partages, chaque partage étant réalisé avec des volumes identiques, noté V, de solutions aqueuse et organique.

Sur les figures 1A et 1B :

- $C_0$ correspond à la concentration totale en soluté(s) mesurée dans les solutions 1 et 2, et la grandeur X extensive serait alors $n_0 = C_0 \times V$.
- $C_{11}$ et $C_{12}$ correspondent respectivement aux concentrations totales dans les deux phases, respectivement aqueuse et organique, issues du premier rang de partages, noté i = 1, des solutions 1 et 2.
- $C_{21}$ et $C_{23}$ correspondent aux concentrations totales dans les deux phases aqueuses issues du deuxième rang de partages, noté i = 2, tandis que
- $C_{22}$ et $C_{24}$ correspondent aux concentrations totales dans les deux phases organiques issues du deuxième rang de partages.

Comme visible sur la figure 1B, les solutés de la solution 2 se répartissent entre phase aqueuse et phase organique différemment du soluté de la solution 1 lors du premier rang de partages. Toutefois, leurs contributions respectives à la grandeur X « s'équilibrent » ($C_{11} = C_{12} = 0,5 \times C_0$) en sorte que l'on obtient, dans le cas de la solution 2, un log D moyen de 0 et, donc, égal à celui obtenu pour la solution 1. Les solutions 1 et 2 ne sont, de ce fait, pas différenciables.

En revanche, les log D obtenus pour les phases issues du deuxième rang de partages de la solution 2, qui sont respectivement égaux à -1 et +1, permettent de différencier cette solution de la solution 1 dont le log D est égal à 0.

C'est ce principe qui est mis à profit dans le procédé de l'invention qui prévoit de soumettre la solution $S_0$ que l'on cherche à caractériser à une séquence de N rangs de partages, N étant un nombre entier supérieur ou égal à 2.

Le premier rang de partages correspond au partage d'un échantillon de la solution $S_0$ que l'on réalise en mélangeant cet échantillon avec un volume d'une solution non miscible à la solution $S_0$ afin que les solutés présents dans

la solution $S_0$ se partagent entre les deux phases formant le mélange, puis, une fois les solutés à l'équilibre, en séparant les deux phases du mélange l'une de l'autre, par exemple par décantation ou centrifugation.

Si la solution $S_0$ est une solution aqueuse, alors la solution non miscible à elle est une solution organique, typiquement un solvant organique non miscible à l'eau, par exemple le n-octanol (que l'on a, de préférence, préalablement hydraté, c'est-à-dire pré-équilibré par contact avec de l'eau).

Si la solution $S_0$ est une solution organique, alors la solution non miscible à elle est une solution aqueuse, typiquement de l'eau, de préférence distillée.

À l'issue du premier rang de partages, deux phases, respectivement aqueuse et organique, sont donc obtenues.

Chaque rang de partages postérieur au premier rang de partages correspond aux partages des phases issues du rang de partages qui le précède, partages que l'on réalise :

- en mélangeant chaque phase aqueuse avec un volume d'une solution organique puis, une fois les solutés à l'équilibre, en séparant le mélange ainsi obtenu en deux phases, respectivement aqueuse et organique, par exemple par décantation ou centrifugation, et
- en mélangeant chaque phase organique avec un volume d'une solution aqueuse puis, une fois les solutés à l'équilibre, en séparant le mélange ainsi obtenu en deux phases, respectivement aqueuse et organique, par exemple par décantation ou centrifugation.

Si la solution $S_0$ est une solution aqueuse, alors :

- la solution organique, qui est utilisée à chacun des rangs de partages postérieurs au premier rang de partages, est la même que celle qui est utilisée au premier rang de partages, tandis que
- la solution aqueuse, qui est utilisée à chacun des rangs de partages postérieurs au premier rang de partages, est une solution aqueuse qui présente, de préférence, une force ionique et un pH proches de ceux de la solution aqueuse $S_0$ et qui comprend, si nécessaire, un sel de fond tel que KCl ou $NaClO_4$ et/ou un tampon pH dans de l'eau, de préférence distillée.

Si la solution $S_0$ est une solution organique, alors :

- la solution aqueuse, qui est utilisée à chacun des rangs de partages postérieurs au premier rang de partages, est la même que celle utilisée au premier rang de partages, tandis que
- la solution organique, qui est utilisée à chacun des rangs de partages postérieurs au premier rang de partages, est soit le même solvant organique que celui que comprend la solution $S_0$ soit, à défaut, un solvant organique non miscible à l'eau, éventuellement préalablement hydraté, tel que le n-octanol.

On obtient ainsi un total de $[2^{(N+1)}-1]$ phases (en comptant la solution $S_0$ que l'on cherche à caractériser) dont $2^N$ phases sont issues du dernier rang de partages.

À titre d'exemple, la figure 2 illustre schématiquement les phases obtenues dans le cas d'une séquence de 3 rangs de partages (N = 3).

Sur cette figure, les phases inscrites dans des rectangles dont les contours en trait plein correspondent à des phases aqueuses tandis que les phases inscrites dans des rectangles dont les contours sont en pointillés correspondent à des phases organiques, la solution $S_0$ pouvant être indifféremment aqueuse ou organique.

Par ailleurs, sur cette figure, toutes les phases sont affectées d'un indice à deux chiffres dont le premier chiffre correspond au rang de partages, noté i, dont elles sont issues et dont le deuxième chiffre correspond à l'ordre, noté j, dans lequel elles se trouvent dans ce rang de partages.

Ainsi, par exemple, la phase $\varphi_{34}$ est la 4ème phase (j = 4) issue du 3ème rang (i = 3) de partages.

Il va de soi que plus N sera grand, plus la caractérisation de la solution $S_0$ sera fine, N étant toutefois limité par la sensibilité de la technique utilisée pour mesurer la grandeur X puisque les valeurs de la grandeur X diminuent au fur et à mesure qu'augmente le nombre de rangs de partages.

C'est la raison pour laquelle N est typiquement compris entre 2 et 20 et, de préférence, entre 3 et 10.

### 3. Mesures de la grandeur X (étape b) du procédé) :

Comme précédemment mentionné, le procédé de l'invention prévoit de mesurer une grandeur X dans toutes les phases, aqueuses et organiques, obtenues au dernier rang N de partage.

La grandeur X peut être toute grandeur permettant de cibler une propriété d'intérêt présentée par les solutés ou certains des solutés de la solution $S_0$ pour autant que cette grandeur soit extensive d'une part, et conservative, d'autre part, c'est-à-dire que :

$$^{j}\sum X_{(ij)} = {}^{j}\sum X_{(i+1,j)}.$$

Ainsi, il peut notamment s'agir :

- d'une quantité de matière (molaire, massique ou autre) ou d'une concentration (ramenée à une quantité de matière en multipliant par les volumes de la solution $S_0$ ou des phases mesurées) telle que le carbone organique total (COT), l'azote total (selon la méthode de Kjeldahl), la demande chimique en oxygène (DCO), la demande biochimique en oxygène (DBO), la matière en suspension (MES), etc.,
- d'une absorbance à une longueur d'onde donnée,
- d'une fluorescence à une longueur d'onde d'excitation ou d'émission donnée,
- d'une radioactivité (comme l'activité du $^{14}$C par exemple),
- d'une teneur en colloïdes, ou encore
- d'une opacité.

Sont ainsi obtenues $2^N$ valeurs de X mesurées qui permettent de définir un vecteur expérimental $\vec{V}$exp qui, dans le cas, par exemple, de la séquence de partages montrée sur la figure 2 est à 8 valeurs, respectivement $X_{31}$, $X_{32}$, $X_{33}$, $X_{34}$, $X_{35}$, $X_{36}$, $X_{37}$ et $X_{38}$, chaque valeur de X étant affectée du même indice que celui affectant la phase, aqueuse ou organique, pour laquelle elle a été obtenue.

Il est à noter que, conformément à l'invention, la grandeur X peut être mesurée dans toutes les phases, aqueuses et organiques, obtenues à chacun des N rangs de partages afin d'améliorer la précision expérimentale. Toutefois, cela n'est pas indispensable, seule la mesure de la grandeur X dans toutes les phases aqueuses et organiques issues du dernier rang N de partages étant nécessaire.

## 4. Traitement mathématique des valeurs de X mesurées (étapes c) et d) du procédé) :

### 4.1. Définition des vecteurs théoriques $\vec{V}$théor($D_k$) :

Pour définir les vecteurs $\vec{V}$théor($D_k$), on commence par calculer, pour toutes les phases aqueuses et organiques obtenues à chacun des N rangs de partages, les valeurs de X attendues pour des solutés présentant y valeurs différentes de D, notées $D_k$, y étant supérieur ou égal à 2.

Ces calculs peuvent être réalisés en appliquant les équations (10a) et (10b) :

$$X_{(i+1,2j-1)}(D_k) = X_{(ij)}(D_k) \times \frac{1}{\left(1 + D_k \times \frac{V_{(ij)org}}{V_{(ij)aq}}\right)} \qquad (10a)$$

$$X_{(i+1,2j)}(D_k) = X_{(ij)}(D_k) \times \frac{D_k \times \frac{V_{(ij)org}}{V_{(ij)aq}}}{\left(1 + D_k \times \frac{V_{(ij)org}}{V_{(ij)aq}}\right)} \qquad (10b)$$

qui ne sont que l'application des équations (9a) et (9b) ci-avant au partage d'une phase ij (c'est-à-dire de rang i et d'ordre j dans ce rang) et une synthèse, d'une part, des équations (1a), (2a) et (3a) ci-avant pour l'équation (10a) et, d'autre part, des équations (1b), (2b) et (3b) ci-avant pour l'équation (10b).

Dans les équations (10a) et (10b) :

$X_{(i+1,2j-1)}(D_k)$ est la valeur de X attendue pour des solutés présentant la valeur $D_k$ et pour la phase aqueuse issue du partage de la phase ij,

$X_{(i+1,2j)}(D_k)$ est la valeur de X attendue pour des solutés présentant la valeur $D_k$ et pour la phase organique issue du partage de la phase ij,

$X_{(ij)}(D_k)$ est la valeur de X attendue pour des solutés présentant la valeur $D_k$ et pour la phase ij,

$V_{(ij)org}$ est le volume de la phase organique soumise au partage ij ou le volume de solution organique $S_{org}$ utilisé pour réaliser le partage de la phase ij (si la phase ij est une phase aqueuse), et

$V_{(ij)aq}$ est le volume de la phase aqueuse soumise au partage ij ou le volume de solution aqueuse $S_{aq}$ utilisé pour réaliser le partage de la phase ij (si la phase ij est une phase organique).

Ainsi, appliquées, par exemple, au partage de la phase aqueuse $\varphi_{21}$ montrée sur la figure 2 au moyen d'un volume de solution organique $S_{org}$, noté $V_{21}(S_{org})$, les équations (10a) et (10b) deviennent respectivement :

$$X_{31}(D_k) = X_{21}(D_k) \times \frac{1}{\left(1 + D_k \times \frac{V_{21}(S_{org})}{V(\varphi_{21})}\right)}$$

$$X_{32}(D_k) = X_{21}(D_k) \times \frac{D_k \times \frac{V_{21}(S_{org})}{V(\varphi_{21})}}{\left(1 + D_k \times \frac{V_{21}(S_{org})}{V(\varphi_{21})}\right)}$$

dans lesquelles :

$D_k$ est tel que précédemment défini,
$X_{31}(D_k)$ est la valeur de X attendue pour des solutés présentant la valeur $D_k$ et pour la phase aqueuse $\varphi_{31}$,
$X_{32}(D_k)$ est la valeur de X attendue pour des solutés présentant la valeur $D_k$ et pour la phase organique $\varphi_{32}$,
$X_{21}(D_k)$ est la valeur de X attendue pour des solutés présentant la valeur $D_k$ et pour la phase aqueuse $\varphi_{21}$,
$V_{21}(S_{org})$ est le volume de solution organique $S_{org}$ utilisé pour réaliser le partage de la phase aqueuse $\varphi_{21}$, et
$V_{21}$ est le volume de la phase aqueuse $\varphi_{21}$ soumise au partage.

Comme précédemment indiqué, les valeurs $D_k$ sont au moins au nombre de deux mais peuvent être plus nombreuses, par exemple au nombre de 3, 4, 5, 6, 7, 8 ou davantage avec :

- lorsqu'elles sont au nombre de 3, par exemple $D_1 = 10^{-1}$ (i.e. $\log D_1 = -1$), $D_2 = 10^0$, (i.e. $\log D_2 = 0$) et $D_3 = 10$ (i.e. $\log D_3 = +1$), ou bien
- lorsqu'elles sont au nombre de 5, par exemple $D_1 = 10^{-2}$ (i.e. $\log D_1 = -2$), $D_2 = 10^{-1}$ (i.e. $\log D_2 = -1$), $D_3 = 10^0$ (i.e. $\log D_3 = 0$), $D_4 = 10$ (i.e. $\log D_4 = +1$) et $D_5 = 10^2$ (i.e. $\log D_5 = +2$),
- etc.

Il est à noter que l'application des équations (10a) et (10b) au partage de la solution $S_0$ nécessite de connaître la valeur de X, notée $X_0$, pour cette solution et, donc, de l'avoir mesurée au préalable.

À partir de ces calculs, il est ensuite possible d'établir, pour le dernier rang N de partages, y vecteurs $\vec{V}$théor($D_k$) chacun de dimension $2^N$ et dont les éléments représentent la contribution théorique des solutés, qui présentent la valeur $D_k$, à la grandeur X mesurée dans les phases aqueuses et organiques issues de ce rang de partages, i.e. le $n^{\text{ième}}$ élément des $\vec{V}$théor($D_k$) est $V_n$théor($D_k$) = $X_n(D_k)/X_0$ (en %).

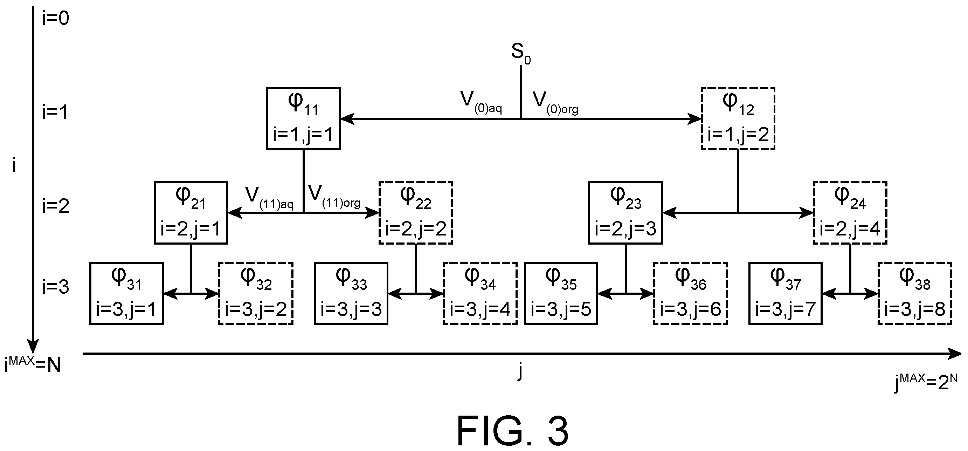

Ainsi, comme illustré à titre d'exemple sur la partie droite de la figure 3, les vecteurs $\vec{V}$théor($D_k$) pour la séquence de partages montrée sur la figure 2, notés $\vec{V}_1(D_1)$, $\vec{V}_2(D_2)$, $\vec{V}_3(D_3)$, ..., $\vec{V}_y(D_y)$, sont chacun à 8 éléments, respectivement $X_{31}$, $X_{32}$, $X_{33}$, $X_{34}$, $X_{35}$, $X_{36}$, $X_{37}$ et $X_{38}$, chaque élément étant affecté, là également, du même indice que celui affectant la phase, aqueuse ou organique, auquel il correspond.

### 4.2. Ajustement paramétrique :

Comme illustré sur la figure 3, une fois le vecteur $\vec{V}$exp et les $\vec{V}$théor($D_k$) établis, une comparaison des données du vecteur Vexp à une combinaison linéaire des vecteurs $\vec{V}_1(D_1)$, $\vec{V}_2(D_2)$, $\vec{V}_3(D_3)$, ..., $\vec{V}_y(D_y)$, permet d'ajuster les paramètres que sont $P_k$ (%) d'une part, et, éventuellement, $D_k$ d'autre part, de sorte que :

$$\vec{V}\text{exp} \approx \Sigma(P_k\,(\%) \times \vec{V}\text{théor}(D_k)).$$

Peut ainsi être obtenu un histogramme tel que celui montré sur la figure 3, y étant, dans le cas de cet histogramme, égal à 8 et l'hydrophilie/lipophilie étant exprimée en $\log D_k$

## 5. Validation du procédé de l'invention :

On prépare un mélange aqueux comprenant 3 solutés étalons de lipophilies (c'est-à-dire de valeurs de log D) différentes, à savoir l'acide benzoïque, la théophylline et le benzimidazole.

Dans les conditions expérimentales utilisées pour cette validation, le log D de l'acide benzoïque est égal à -1,24, celui de la théophylline est égal à -0,02, tandis que celui du benzimidazole est égal à +1,34.

La quantité totale de moles des solutés dans le mélange (i.e. $n_0$) est d'environ $5 \times 10^{-4}$ moles. Le pH du mélange est d'environ 7,4 (tampon phosphate : PBS).

Une séquence de 3 rangs de partages - telle qu'illustrée sur la figure 2 - est appliquée au mélange en utilisant du n-octanol hydraté pour le partage du mélange aqueux et celui des phases aqueuses issues des partages de rangs 1 et 2 (i.e. les phases $\varphi 11$, $\varphi 21$ et $\varphi 23$), et une solution aqueuse comprenant du tampon PBS de pH 7 dans de l'eau distillée pour le partage des phases organiques issues des partages de rangs 1 et 2 (i.e. les phases $\varphi 12$, $\varphi 22$ et $\varphi 24$). Le rapport $V_{org}/V_{aq}$ utilisé est de l'ordre de 1/4.

La concentration de chaque soluté dans les phases issues des partages de rangs 1, 2 et 3 (i.e. les phases $\varphi 11$ à $\varphi 38$) est suivie par spectroscopie UV-visible mais uniquement dans le but de contrôler le bon déroulement de l'expérimentation.

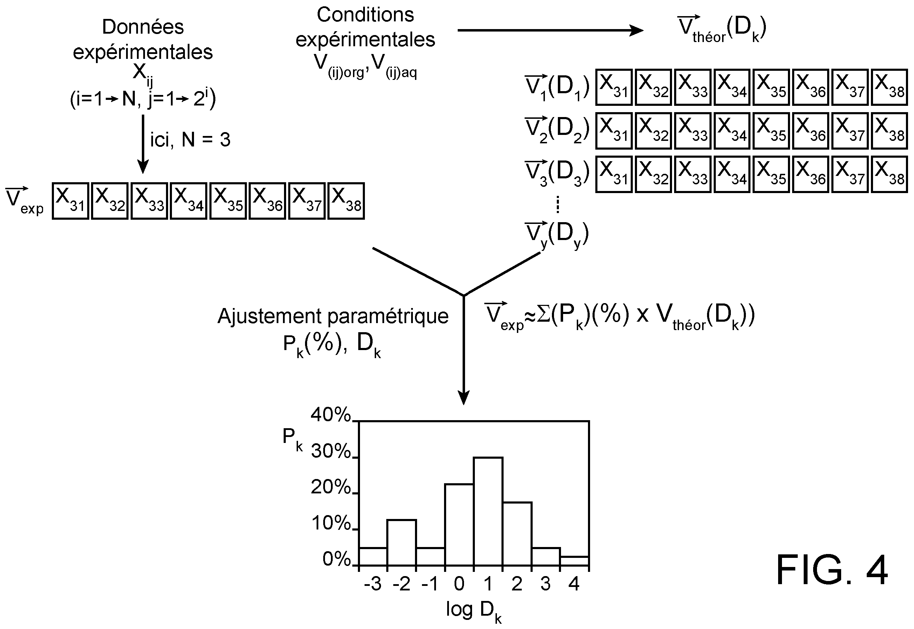

Comme le montre la partie gauche de la figure 4, qui montre les coefficients de partage, exprimés en log D, tels que vérifiés expérimentalement à chacun des rangs de partages et pour chacun des solutés, la théophylline et le benzimidazole se partagent entre phases aqueuses et phases organiques de manière suffisamment homogène pour que leur concentration puisse être mesurée dans toutes les phases (à l'exception du benzimidazole dans la phase $\varphi 31$). Par contre, de par son caractère hydrophile, l'acide benzoïque est extrait en quasi-totalité dans les phases aqueuses : $\varphi 11$ puis $\varphi 21$ et $\varphi 31$. La mesure de son coefficient de partage n'est donc possible qu'à partir de la séparation de ces trois dernières phases. Les coefficients de partage mesurés individuellement pour les trois composés sont proches des valeurs théoriques indiquées en pointillés, attestant du bon déroulement des expériences successives.

Les résultats obtenus en appliquant le traitement mathématique décrit au point 4 ci-avant sont illustrés dans le tableau ci-après ainsi que sur la partie droite de la figure 4.

Dans ce tableau, sont présentés :
- les log D des solutés tels que vérifiés expérimentalement, notés log D expérimentaux,
- le nombre de moles de chacun des solutés dans le mélange de départ tel que vérifié expérimentalement, noté $n_{mélange}$,
- les log D utilisés pour définir les vecteurs théoriques, notés log D cibles, et
- le nombre de moles obtenu pour chaque soluté noté, $n_{résultat}$, et obtenu par ajustement de la quantité totale des trois solutés mesurée dans chaque phase.

|  | Acide benzoïque | Théophylline | Benzimidazole | $n_{tot}$ (moles) |
|---|---|---|---|---|
| log D expérimentaux | -1,24 | 0,01 | 1,33 |  |
| $n_{mélange}$ (moles) | $2,05.10^{-4}$ | $4,36.10^{-5}$ | $2,12.10^{-4}$ | $5.10^{-4}$ |
| log D cibles | -1 | 0 | 1 |  |
| $n_{résultat}$ (moles) | $1,86.10^{-4}$ | $< 10^{-5}$ | $1,66.10^{-4}$ | $3,5.10^{-4}$ |

Dans la partie droite de la figure 4, les résultats sont exprimés en pourcentages en termes de quantité de matière d'un soluté (nX) rapportée à la quantité totale de matière des solutés dans le mélange ($n_{tot}$), notés P(X)%, en fonction du coefficient de partage, exprimé en log D.

Le traitement mathématique utilise uniquement la valeur de la quantité totale (en moles) des solutés dans les phases du dernier rang de partages, qui est rapportée à la quantité totale de ces solutés dans le mélange de départ.

Différents ajustements paramétriques sont testés avec un nombre variable de vecteurs théoriques (3 ou 5) ou des valeurs de log D différentes. Dans chaque cas, les données obtenues par le traitement mathématique (indiquées par des signes) sont relativement proches des valeurs attendues pour ce mélange connu (indiquées par des ronds).

Ce type de résultats a été reproduit avec différents rapports de quantités entre les solutés, différentes gammes de concentrations, d'autres solutés, et différents types de grandeur X ($^{14}$C, COT, etc.), ce qui démontre une faisabilité générale pour le procédé de l'invention.

## 6. Évaluation du degré de pureté d'un bisphénol A marqué par du $^{14}$C et caractérisation d'une impureté :

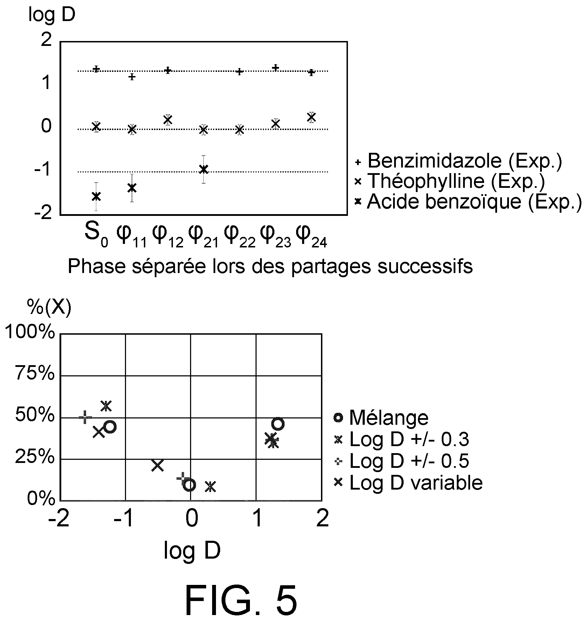

On se réfère à présent à la figure 5 qui illustre un exemple de mise en œuvre du procédé de l'invention pour l'évaluation du degré de pureté d'une substance de synthèse et la caractérisation de la quantité et de la lipophilie d'une impureté que comprend cette substance, qui est l'une des applications potentielles de ce procédé.

Cet exemple a été réalisé avec une source commerciale de bisphénol A (BPA) marqué au $^{14}$C, plus simplement dénommé ci-après $^{14}$C-BPA.

La grandeur X est donc l'activité en $^{14}$C que l'on mesure par scintillation liquide.

Comme le montre la figure 5, la mesure de l'activité en $^{14}$C à l'issue d'un unique partage (N = 1) de la source commerciale de $^{14}$C-BPA, réalisé avec de l'eau distillée et du *n*-octanol, fournit un log D, noté log $D^{EXP}$ (source $^{14}$C-BPA), sensiblement égal à 2,1 (trait plein) et, donc, très différent du log D que présente théoriquement le BPA, noté log $D^{THÉOR}$ (BPA) et qui est de 3,32 (trait pointillé).

Malgré une pureté élevée de la source commerciale ($\approx$ 99%), une différence significative est mesurée vis-à-vis de la lipophilie théorique du BPA du fait de la présence d'une impureté aux propriétés très différentes de celles du BPA : log D (impureté) << log D (BPA).

Un second rang de partages (N = 2) est réalisé sur les phases $\varphi_{11}$ et $\varphi_{12}$ issues du premier partage (i = 1).

L'activité en $^{14}$C est mesurée sur les 4 phases, $\varphi_{21}$ à $\varphi_{24}$, issues de ce second rang de partages. Les résultats de ces mesures sont comparés à l'aide du traitement mathématique décrit au point 4, aux résultats théoriquement obtenus pour un mélange de deux espèces inconnues de lipophilie D1 et D2 différentes.

Comme le montre la figure 5, ont ainsi été mises en évidence dès le second rang de partages :

- d'une part, une quantité d'environ 99% en activité en $^{14}$C, correspondant au de $^{14}$C-BPA avec un log D, noté log $D^{EXP}$ (BPA), d'environ 3 et, donc, proche du log D que présente théoriquement le BPA, et
- d'autre part, une quantité d'environ 1,3% en activité en $^{14}$C, correspondant à une impureté présentant un log D, noté log $D^{EXP}$ (impureté), sensiblement égal à -0,5.

On notera que ces résultats obtenus « en aveugle » sont en accord avec le taux d'impureté estimé par le fournisseur, qui est de l'ordre de 1%, et la présence dans la source commerciale de $^{14}$C-BPA de possibles résidus d'acétone marquée au [14]C. En effet, comme montré sur la figure 5, le BPA est synthétisé par réaction de deux molécules de phénol avec une molécule d'acétone dont le log D théorique, noté log $D^{THÉOR}$ (acétone), est sensiblement égal à -0,24.

## Références citées

Mondeguer et al., Spectra Analyse 2012, 284, 25-33
Fischer et al., Trends in Analytical Chemistry 2021, 136, 116188
Milman et Zhurkovich, Trends in Analytical Chemistry 2017, 97, 179-187

## Revendications

1. Procédé de caractérisation d'une solution $S_0$ comprenant une pluralité de solutés, qui comprend au moins les étapes consistant à :

   a) soumettre la solution $S_0$ à une séquence de N rangs i de partages, i allant de 1 à N et N étant supérieur ou égal à 2, les rangs i de partages comprenant :

      - pour i=1, le mélange d'un échantillon de la solution $S_0$ avec un volume d'une solution organique $S_{org}$ ou aqueuse $S_{aq}$ non miscible à la solution $S_0$ pour obtenir, après séparation du mélange en deux phases, une phase aqueuse $\varphi(i=1,aq)$ et une phase organique $\varphi(i=1,org)$ ; et
      - pour i=2 à N :

         * le mélange de chaque phase aqueuse $\varphi(i-1,aq)$ avec un volume de la solution $S_{org}$ pour obtenir, après séparation du mélange en deux phases, une phase aqueuse $\varphi(i,aq)$ et une phase organique $\varphi(i,org)$ ;
         * le mélange de chaque phase organique $\varphi(i-1,org)$ avec un volume de la solution $S_{aq}$ pour obtenir, après séparation du mélange en deux phases, une phase aqueuse $\varphi(i,aq)$ et une phase organique $\varphi(i,org)$ ;

      b) soumettre toutes les phases aqueuses et organiques issues du rang N à une mesure d'une grandeur X extensive et conservative pour obtenir un vecteur expérimental $\vec{V}exp$ comprenant $2^N$ valeurs de X mesurées ;
      c) définir des vecteurs théoriques $\vec{V}théor(D_k)$ pour y valeurs $D_k$ de coefficient de partage avec y supérieur ou égal à 2 et k allant de 1 à y, chaque vecteur théorique comprenant $2^N$ valeurs de X calculées pour les phases aqueuses et organiques issues du rang N et pour l'une des y valeurs $D_k$ ;

d) réaliser un ajustement paramétrique entre les vecteurs $\vec{V}$théor($D_k$) et le vecteur $\vec{V}$exp pour obtenir une répartition des solutés de la solution $S_0$, qui contribuent aux $2^N$ valeurs de X mesurées, en fonction des y valeurs $D_k$.

2. Procédé selon la revendication 1, dans lequel N est compris entre 2 et 20, de préférence, entre 3 et 10.

3. Procédé selon la revendication 1 ou la revendication 2, dans lequel la solution $S_0$ est une solution aqueuse.

4. Procédé selon la revendication 3, dans lequel la solution $S_{org}$ est un solvant organique non miscible à l'eau et la solution $S_{aq}$ est de l'eau, de préférence distillée, comprenant éventuellement un sel de fond et/ou un tampon pH.

5. Procédé selon la revendication 1 ou la revendication 2, dans lequel la solution $S_0$ est une solution organique.

6. Procédé selon la revendication 5, dans lequel la solution $S_{aq}$ est de l'eau, de préférence distillée, et la solution $S_{org}$ est un solvant organique non miscible à l'eau.

7. Procédé selon la revendication 4 ou la revendication 6, dans lequel le solvant organique est un alcool en C8 à C12, un alcane en C5 à C16, le cyclohexane, un hydrocarbure aromatique, de préférence le n-octanol.

8. Procédé selon l'une quelconque des revendications 1 à 7, dans lequel la grandeur X est une quantité de matière, une concentration, une absorbance, une fluorescence, une radioactivité ou une opacité.

9. Procédé selon l'une quelconque des revendications 1 à 8, dans lequel chaque vecteur $\vec{V}$théor($D_k$) est défini en appliquant :

- au partage de l'échantillon de la solution $S_0$, les équations (1a) et (1b) suivantes :

$$X_{(i=1,aq)}(D_k) = X_0 \times \frac{1}{\left(1 + D_k \times \frac{V_{(0,org)}}{V_{(0,aq)}}\right)} \qquad (1a)$$

$$X_{(i=1,org)}(D_k) = X_0 \times \frac{D_k \times \frac{V_{(0,org)}}{V_{(0,aq)}}}{\left(1 + D_k \times \frac{V_{(0,org)}}{V_{(0,aq)}}\right)} \qquad (1b)$$

- aux partages des phases $\varphi(i,aq)$, les équations (2a) et (2b) suivantes :

$$X_{(i+1,aq)}(D_k) = X_{(i,aq)}(D_k) \times \frac{1}{\left(1 + D_k \times \frac{V_{(i,org)}}{V_{(i,aq)}}\right)} \qquad (2a)$$

$$X_{(i+1,org)}(D_k) = X_{(i,aq)}(D_k) \times \frac{D_k \times \frac{V_{(i,org)}}{V_{(i,aq)}}}{\left(1 + D_k \times \frac{V_{(i,org)}}{V_{(i,aq)}}\right)} \qquad (2b)$$

- aux partages des phases $\varphi(i,org)$, les équations (3a) et (3b) suivantes :

$$X_{(i+1,aq)}(D_k) = X_{(i,org)}(D_k) \times \frac{1}{\left(1 + D_k \times \frac{V_{(i,org)}}{V_{(i,aq)}}\right)} \qquad (3a)$$

$$X_{(i+1,org)}(D_k) = X_{(i,org)}(D_k) \times \frac{D_k \times \frac{V_{(i,org)}}{V_{(i,aq)}}}{\left(1 + D_k \times \frac{V_{(i,org)}}{V_{(i,aq)}}\right)} \qquad (3b)$$

dans lesquelles :

$X_{(i=1,aq)}$ est la valeur de X attendue pour des solutés présentant la valeur $D_k$ et pour la phase φ(i=1,aq),
$X_{(i=1,org)}$ est la valeur de X attendue pour des solutés présentant la valeur $D_k$ et pour la phase φ(i=1,org),
$X_0$ est la valeur de X mesurée dans la solution $S_0$,
$V_{(0,org)}$ est le volume de l'échantillon de solution $S_0$ si la solution $S_0$ est une solution organique ou le volume de solution $S_{org}$ utilisé pour partager l'échantillon de la solution $S_0$ si la solution $S_0$ est une solution aqueuse,
$V_{(0,aq)}$ est le volume de l'échantillon de solution $S_0$ si la solution $S_0$ est une solution aqueuse ou le volume de solution $S_{aq}$ utilisé pour partager l'échantillon de la solution $S_0$ si la solution $S_0$ est une solution organique,
$X_{(i+1,aq)}$ est la valeur de X attendue pour des solutés présentant la valeur $D_k$ et pour une phase φ(i+1,aq) issue du partage d'une phase φ(i,aq) ou d'une phase φ(i,org),
$X_{(i+1,org)}$ est la valeur de X attendue pour des solutés présentant la valeur $D_k$ et pour une phase φ(i+1,org) issue du partage d'une phase φ(i,aq) ou d'une phase φ(i,org),
$X_{(i,aq)}$ est la valeur de X attendue pour des solutés présentant la valeur $D_k$ et pour la phase φ(i,aq) partagée,
$X_{(i,org)}$ est la valeur de X attendue pour des solutés présentant la valeur $D_k$ et pour la phase φ(i,org) partagée,
V(i,aq) est le volume de la phase φ(i,aq) partagée ou le volume de solution $S_{aq}$ utilisé pour partager la phase φ(i,org),
V(i,org) est le volume de la phase φ(i,org) partagée ou le volume de solution $S_{org}$ utilisé pour partager la phase φ(i,aq),

moyennant quoi on obtient pour les phases aqueuses et organiques issues du rang N, $2^N$ valeurs de X attendues pour des solutés présentant la valeur $D_k$.

10. Procédé selon l'une quelconque des revendications 1 à 9, dans lequel l'ajustement paramétrique comprend une détermination d'un ensemble de pourcentages $P_k$ en tant que coefficients de pondération tels que :

$$\vec{V}\text{exp} \approx \Sigma(P_k\ (\%) \times \vec{V}\text{théor}(D_k)).$$

11. Procédé selon la revendication 10, dans lequel l'ajustement comprend de plus un ajustement des valeurs $D_k$.

12. Procédé selon l'une quelconque des revendications 1 à 11, dans lequel la répartition des solutés de la solution $S_0$, qui contribuent aux $2^N$ valeurs de X mesurées, en fonction des y valeurs $D_k$ est exprimée sous forme d'un histogramme.

13. Procédé selon l'une quelconque des revendications 1 à 12, dans lequel les solutés sont des solutés organiques.

**Patentansprüche**

1. Verfahren zur Charakterisierung einer Lösung $S_0$, umfassend eine Vielzahl von gelösten Stoffen, das mindestens die Schritte umfasst, die aus Folgendem bestehen:

a) Aussetzen der Lösung $S_0$ einer Sequenz von N Teilungsreihen i, wobei i von 1 bis N reicht und N größer oder gleich 2 ist, wobei die Teilungsreihen i Folgendes umfassen:

- für i=1, das Mischen einer Probe der Lösung $S_0$ mit einem Volumen einer organischen Lösung $S_{org}$ oder wässrigen Lösung $S_{aq}$, die nicht mit der Lösung $S_0$ mischbar ist, um nach dem Trennen der Mischung in zwei Phasen eine wässrige Phase $\varphi(i=1,aq)$ und eine organische Phase $\varphi(i=1,org)$ zu erhalten; und
- für i=2 bis N:

* Mischen jeder wässrigen Phase $\varphi(i-1,aq)$ mit einem Volumen der Lösung $S_{org}$, um nach dem Trennen der Mischung in zwei Phasen eine wässrige Phase $\varphi(i,aq)$ und eine organische Phase $\varphi(i,org)$ zu erhalten;
* das Mischen jeder organischen Phase $\varphi(i-1,org)$ mit einem Volumen der Lösung $S_{aq}$, um nach dem Trennen des Gemischs in zwei Phasen eine wässrige Phase $\varphi(i,aq)$ und eine organische Phase $\varphi(i,org)$ zu erhalten;

b) Aussetzen aller wässrigen und organischen Phasen aus der Reihe N einer Messung einer umfangreichen und konservativen Größe X, um einen experimentellen Vektor $\vec{V}exp$ zu erhalten, der $2^N$ gemessene X-Werte umfasst;
c) Definieren von theoretischen Vektoren $\vec{V}theor(D_k)$ für y $D_k$-Werte des Teilungskoeffizienten, wobei y größer oder gleich 2 ist und k von 1 bis y reicht, wobei jeder theoretische Vektor $2^N$ Werte von X umfasst, die für die wässrigen und organischen Phasen berechnet werden, die aus der Reihe N stammen, und für einen der y $D_k$-Werte;
d) Durchführen einer parametrischen Anpassung zwischen den Vektoren $\vec{V}theor(D_k)$ und dem Vektor $\vec{V}exp$, um eine Verteilung der gelösten Stoffe der Lösung $S_0$ zu erhalten, die zu den $2^N$ gemessenen X-Werten in Abhängigkeit von den y $D_k$-Werten beitragen.

2. Verfahren nach Anspruch 1, wobei N zwischen 2 und 20, vorzugsweise zwischen 3 und 10 liegt.

3. Verfahren nach Anspruch 1 oder Anspruch 2, wobei die Lösung $S_0$ eine wässrige Lösung ist.

4. Verfahren nach Anspruch 3, wobei die Lösung $S_{org}$ ein nicht mit Wasser mischbares organisches Lösungsmittel ist und die Lösung $S_{aq}$ Wasser ist, das vorzugsweise destilliert ist und gegebenenfalls ein Grundsalz und/oder einen pH-Puffer umfasst.

5. Verfahren nach Anspruch 1 oder Anspruch 2, wobei die Lösung $S_0$ eine organische Lösung ist.

6. Verfahren nach Anspruch 5, wobei die Lösung $S_{aq}$ Wasser, vorzugsweise destilliert, und die Lösung $S_{org}$ ein nicht mit Wasser mischbares organisches Lösungsmittel ist.

7. Verfahren nach Anspruch 4 oder Anspruch 6, wobei das organische Lösungsmittel ein C8- bis C12-Alkohol, ein C5- bis C16-Alkan, Cyclohexan, ein aromatischer Kohlenwasserstoff, vorzugsweise n-Octanol ist.

8. Verfahren nach einem der Ansprüche 1 bis 7, wobei die Größe X eine Stoffmenge, eine Konzentration, eine Absorption, eine Fluoreszenz, eine Radioaktivität oder eine Trübung ist.

9. Verfahren nach einem der Ansprüche 1 bis 8, wobei jeder Vektor $\vec{V}$ theor($D_k$) durch Anwenden von Folgendem definiert ist:

- durch Teilung der Probe der Lösung $S_0$ folgende Gleichungen (1a) und (1b):

$$X_{(i=1,aq)}(D_k) = X_0 \times \frac{1}{\left(1 + D_k \times \frac{V_{(0,org)}}{V_{(0,aq)}}\right)} \ (1a)$$

$$X_{(i=1,org)}(D_k) = X_0 \times \frac{D_k \times \frac{V_{(0,org)}}{V_{(0,aq)}}}{\left(1 + D_k \times \frac{V_{(0,org)}}{V_{(0,aq)}}\right)} \ (1b)$$

- durch Teilungen der Phasen $\varphi(i,aq)$ die folgenden Gleichungen (2a) und (2b):

$$X_{(i+1,aq)}(D_k) = X_{(i,aq)}(D_k) \times \frac{1}{\left(1 + D_k \times \frac{V_{(i,org)}}{V_{(i,aq)}}\right)} \quad (2a)$$

$$X_{(i+1,org)}(D_k) = X_{(i,aq)}(D_k) \times \frac{D_k \times \frac{V_{(i,org)}}{V_{(i,aq)}}}{\left(1 + D_k \times \frac{V_{(i,org)}}{V_{(i,aq)}}\right)} \quad (2b)$$

- durch Teilungen der Phasen φ(i,org) die folgenden Gleichungen (3a) und (3b):

$$X_{(i+1,aq)}(D_k) = X_{(i,org)}(D_k) \times \frac{1}{\left(1 + D_k \times \frac{V_{(i,org)}}{V_{(i,aq)}}\right)} \quad (3a)$$

$$X_{(i+1,org)}(D_k) = X_{(i,org)}(D_k) \times \frac{D_k \times \frac{V_{(i,org)}}{V_{(i,aq)}}}{\left(1 + D_k \times \frac{V_{(i,org)}}{V_{(i,aq)}}\right)} \quad (3b)$$

in denen:

$X_{(i=1,aq)}$ der erwartete Wert von X für gelöste Stoffe mit dem Wert $D_k$ und für die Phase φ(i=1,aq) ist,
$X_{(i=1,org)}$ der erwartete Wert von X für gelöste Stoffe mit dem Wert $D_k$ und für die Phase φ(i=1,org) ist,
$X_0$ der in Lösung $S_0$ gemessene Wert von X ist,
$V_{(0,org)}$ das Volumen der Probe der Lösung $S_0$ ist, wenn die Lösung $S_0$ eine organische Lösung ist, oder das Volumen der Lösung $S_{org}$, das verwendet wird, um die Probe der Lösung $S_0$ zu teilen, wenn die Lösung $S_0$ eine wässrige Lösung ist,
$V_{(0,aq)}$ das Volumen der Probe der Lösung $S_0$ ist, wenn die Lösung $S_0$ eine wässrige Lösung ist, oder das Volumen der Lösung $S_{aq}$, das verwendet wird, um die Probe der Lösung $S_0$ zu teilen, wenn die Lösung $S_0$ eine organische Lösung ist,
$X_{(i+1,aq)}$ der erwartete Wert von X für gelöste Stoffe mit dem Wert $D_k$ und für eine Phase φ(i+1,aq) aus der Aufteilung einer Phase φ(i,aq) oder einer Phase φ(i,org) ist,
$X_{(i+1,org)}$ der erwartete Wert von X für gelöste Stoffe mit dem Wert $D_k$ und für eine Phase φ(i+1,org) aus der Aufteilung einer Phase φ(i,aq) oder einer Phase φ(i,org) ist,
$X_{(i,aq)}$ der erwartete Wert von X für gelöste Stoffe mit dem Wert $D_k$ und für die geteilte Phase φ(i,aq) ist,
$X_{(i,org)}$ der erwartete Wert von X für gelöste Stoffe mit dem Wert $D_k$ und für die geteilte Phase φ(i,org) ist,
V(i,aq) das Volumen der geteilten φ(i,aq)-Phase oder das Volumen der Lösung $S_{aq}$ ist, das verwendet wird, um die φ(i,org)-Phase zu teilen,
V(i,org) das Volumen der geteilten φ(i,org)-Phase oder das Volumen der Lösung $S_{org}$ ist, das verwendet wird, um die φ(i,aq)-Phase zu teilen,
womit man für wässrige und organische Phasen aus dem Rang N, $2^N$ erwartete X-Werte für gelöste Stoffe erhält, die den Wert $D_k$ besitzen.

**10.** Verfahren nach einem der Ansprüche 1 bis 9, wobei die parametrische Anpassung eine Bestimmung einer Reihe von Prozentsätzen $P_k$ als Gewichtungskoeffizienten umfasst, wie z. B.:

$$\vec{V} \exp \approx \sum \left( P_k(\%) \times \vec{V} \operatorname{theor}(D_k) \right).$$

**11.** Verfahren nach Anspruch 10, wobei die Anpassung ferner eine Anpassung der $D_k$-Werte umfasst.

**12.** Verfahren nach einem der Ansprüche 1 bis 11, wobei die Verteilung der gelösten Stoffe der Lösung $S_0$, die zu den $2^N$ gemessenen X-Werten beitragen, in Abhängigkeit von den y $D_k$-Werten in Form eines Histogramms ausgedrückt wird.

**13.** Verfahren nach einem der Ansprüche 1 bis 12, wobei die gelösten Stoffe organische gelöste Stoffe sind.

**Claims**

**1.** A method for characterising a solution $S_0$ comprising a plurality of solutes, which comprises at least the steps consisting in:

a) subjecting the solution $S_0$ to a sequence of N rows i of partitions, i ranging from 1 to N and N being greater than or equal to 2, the rows i of partitions comprising:

- for i=1, a mixture of a sample of the solution $S_0$ with a volume of an organic $S_{org}$ or aqueous $S_{aq}$ solution immiscible with the solution $S_0$ to obtain, after separation of the mixture into two phases, an aqueous phase $\varphi(i=1,aq)$ and an organic phase $\varphi(i=1,org)$; and
- for i=2 to N:

* the mixture of each aqueous phase $\varphi(i-1,aq)$ with a volume of the solution $S_{org}$ to obtain, after separation of the mixture into two phases, an aqueous phase $\varphi(i,aq)$ and an organic phase $\varphi(i,org)$;
* the mixture of each organic phase $\varphi(i-1,org)$ with a volume of the solution $S_{aq}$ to obtain, after separation of the mixture into two phases, an aqueous phase $\varphi(i,aq)$ and an organic phase $\varphi(i,org)$;

b) subjecting all the aqueous and organic phases obtained at the row N to a measurement of an extensive and conservative quantity X to obtain an experimental vector $\vec{V}exp$ comprising $2^N$ measured values of X;

c) defining theoretical vectors $\vec{V}theor(D_k)$ for y partition coefficient values $D_k$, with y greater than or equal to 2 and k ranging from 1 to y, each theoretical vector comprising $2^N$ values of X calculated for the aqueous and organic phases obtained at the row N and for one of the y values $D_k$;

d) carrying out a parametric adjustment between the vectors $\vec{V}theor(D_k)$ and the vector $\vec{V}exp$ to obtain a distribution of the solutes of the solution $S_0$, which contribute to the $2^N$ measured values of X, as a function of to the y values $D_k$.

**2.** The method of claim 1, wherein N is between 2 and 20, preferably between 3 et 10.

**3.** The method of claim 1 or 2, wherein the solution $S_0$ is an aqueous solution.

**4.** The method of claim 3, wherein the solution $S_{org}$ is an organic solvent immiscible in water and the solution $S_{aq}$ is water, preferably distilled water, optionally comprising a ground salt and/or a pH buffer.

**5.** The method of claim 1 or 2, wherein the solution $S_0$ is an organic solution.

**6.** The method of claim 5, wherein the solution $S_{aq}$ is water, preferably distilled water, and the solution $S_{org}$ is an organic solvent immiscible in water.

**7.** The method of claim 4 or 6, wherein the organic solvent is a C8 to C12 alcohol, a C5 to C16 alkane, cyclohexane or an aromatic hydrocarbon, preferably n-octanol.

**8.** The method of any of claims 1 to 7, wherein the quantity X is an amount of substance, a concentration, an absorbance, a fluorescence, a radioactivity or an opacity.

**9.** The method of any of claims 1 to 8, wherein each theoretical vector $\vec{V}theor(D_k)$ is defined by applying:

- to the partition of the sample of the solution $S_0$, the equations (1a) and (1b):

$$X_{(i=1,aq)}(D_k) = X_0 \times \frac{1}{\left(1 + D_k \times \frac{V_{(0,org)}}{V_{(0,aq)}}\right)} \qquad (1a)$$

$$X_{(i=1,org)}(D_k) = X_0 \times \frac{D_k \times \frac{V_{(0,org)}}{V_{(0,aq)}}}{\left(1 + D_k \times \frac{V_{(0,org)}}{V_{(0,aq)}}\right)} \qquad (1b)$$

- to the partitions of the phases $\varphi(i,aq)$, the equations (2a) and (2b):

$$X_{(i+1,aq)}(D_k) = X_{(i,aq)}(D_k) \times \frac{1}{\left(1 + D_k \times \frac{V_{(i,org)}}{V_{(i,aq)}}\right)} \qquad (2a)$$

$$X_{(i+1,org)}(D_k) = X_{(i,aq)}(D_k) \times \frac{D_k \times \frac{V_{(i,org)}}{V_{(i,aq)}}}{\left(1 + D_k \times \frac{V_{(i,org)}}{V_{(i,aq)}}\right)} \qquad (2b)$$

- to the partitions of the phases $\varphi(i,org)$, the equations (3a) and (3b):

$$X_{(i+1,aq)}(D_k) = X_{(i,org)}(D_k) \times \frac{1}{\left(1 + D_k \times \frac{V_{(i,org)}}{V_{(i,aq)}}\right)} \qquad (3a)$$

$$X_{(i+1,org)}(D_k) = X_{(i,org)}(D_k) \times \frac{D_k \times \frac{V_{(i,org)}}{V_{(i,aq)}}}{\left(1 + D_k \times \frac{V_{(i,org)}}{V_{(i,aq)}}\right)} \qquad (3b)$$

where:

$X_{(i=1,aq)}$ is the expected value of X for solutes having the value $D_k$ and for the phase $\varphi(i=1,aq)$,
$X_{(i=1,org)}$ is the expected value of X for solutes having the value $D_k$ and for the phase $\varphi(i=1,org)$,
$X_0$ is the value of X measured in the solution $S_0$,
$V_{(o,org)}$ is the volume of the sample of solution $S_0$ if the solution $S_0$ is an organic solution or the volume of solution $S_{org}$ used to partition the sample of the solution $S_0$ if the solution $S_0$ is an aqueous solution,
$V_{(o,aq)}$ is the volume of the sample of solution $S_0$ if the solution $S_0$ is an aqueous solution or the volume of solution $S_{aq}$ used to partition the sample of the solution $S_0$ if the solution $S_0$ is an organic solution,
$X_{(i+1,aq)}$ is the expected value of X for solutes having the value $D_k$ and for a phase $\varphi(i+1,aq)$ issued from the partition of a phase $\varphi(i,aq)$ or of a phase $\varphi(i,org)$,
$X_{(i+1,org)}$ is the expected value of X for solutes having the value $D_k$ and for a phase $\varphi(i+1,org)$ issued from the partition of a phase $\varphi(i,aq)$ or of a phase $\varphi(i,org)$,
$X_{(i,aq)}$ is the expected value of X for solutes having the value $D_k$ and for the partitioned phase $\varphi(i,aq)$,
$X_{(i,org)}$ is the expected value of X for solutes having the value $D_k$ and for the partitioned phase $\varphi(i,org)$,
$V(i,aq)$ is the volume of the partitioned phase $\varphi(i,aq)$ or the volume of solution $S_{aq}$ used to partition the phase $\varphi(i,org)$,
$V(i,org)$ is the volume of the partitioned phase $\varphi(i,org)$ or the volume of solution $S_{org}$ used to partition the phase $\varphi(i,aq)$,

whereby, for the aqueous and organic phases issued from the row N, $2^N$ expected values of X are obtained for solutes having the value $D_k$.

**10.** The method of any of claims 1 to 9, wherein the parametric adjustment comprises determining a set of percentages $P_k$

as weighting coefficients such that:

$$\vec{V}\exp \approx \Sigma(P_k\,(\%) \times \vec{V}\text{théor}(D_k)).$$

11. The method of claim 10, wherein the adjustment also comprises adjusting the values $D_k$.

12. The method of any of claims 1 to 11, wherein the distribution of the solutes of the solution $S_0$, which contribute to the $2^N$ measured values of X, as a function of the y values $D_k$, is expressed in the form of a histogram.

13. The method of any of claims 1 to 12, wherein the solutes are organic solutes.

FIG. 1A

| Partages | Solution 1 | | Solution 2 | |
|---|---|---|---|---|
| i = 1 | $C_{11} = C_{12} = 0{,}5 \times C_0$ <br> **log D = 0** | | $C_{11} = C_{12} = 0{,}5 \times C_0$ <br> **log D = 0** | |
| i = 2 | $C_{21} = C_{22}$ <br> **log D = 0** | $C_{23} = C_{24}$ <br> **log D = 0** | $C_{21} \approx 10 \times C_{22}$ <br> **log D ≈ -1** | $C_{23} \approx C_{24}/10$ <br> **log D ≈ +1** |

FIG. 1B

FIG. 3

FIG. 4

FIG. 5

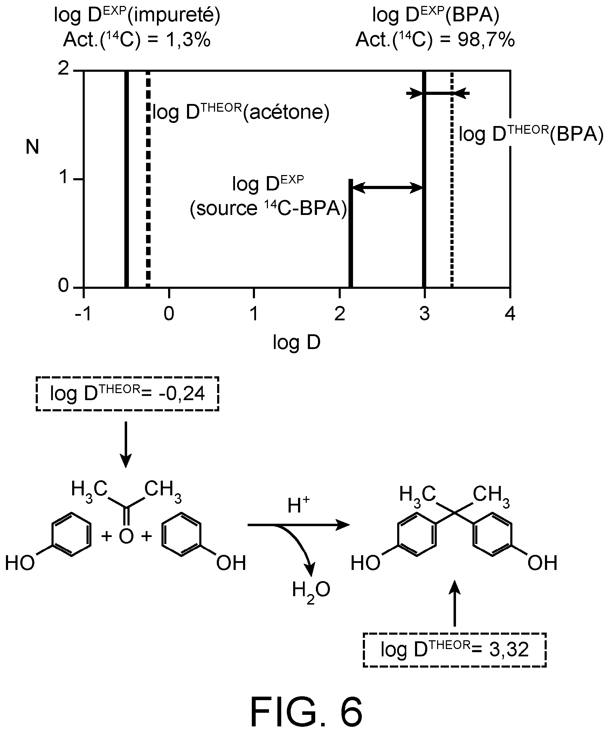

FIG. 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MONDEGUER et al.** *Spectra Analyse*, 2012, vol. 284, 25-33
- **FISCHER et al.** *Trends in Analytical Chemistry*, 2021, vol. 136, 116188

- **ZHURKOVICH**. *Trends in Analytical Chemistry*, 2017, vol. 97, 179-187
- **MILMAN**. *Trends in Analytical Chemistry*, 2017, vol. 97, 179-187